(19) Europäisches Patentamt
European Patent Office
Office européen des brevets

(11) Publication number: **0 080 721**
**A2**

# (12) EUROPEAN PATENT APPLICATION

(21) Application number: 82111004.6

(22) Date of filing: 29.11.82

(51) Int. Cl.³: C 07 C 87/28
C 07 C 87/29, C 07 C 91/26
C 07 D 295/02, C 07 D 295/06
C 07 D 295/08, C 07 D 213/38
C 07 D 307/52, C 07 D 333/20
A 61 K 31/135, A 61 K 31/34

(30) Priority: 30.11.81 JP 193312/81

(43) Date of publication of application:
08.06.83 Bulletin 83/23

(84) Designated Contracting States:
AT BE CH DE FR GB IT LI NL SE

(71) Applicant: SUMITOMO CHEMICAL COMPANY, LIMITED
15 Kitahama 5-chome Higashi-ku
Osaka-shi Osaka-fu(JP)

(72) Inventor: Kawakami, Hajime
14-7, Mefu 2-chome
Takarazuka Hyogo(JP)

(72) Inventor: Ono, Keiichi
5-3-530, Higashiawaji 1-chome Higashiyodogawa-ku
Osaka, Osaka(JP)

(74) Representative: Vossius Vossius Tauchner Heunemann Rauh
Siebertstrasse 4 P.O. Box 86 07 67
D-8000 München 86(DE)

## (54) Diphenylalkanoamine derivatives, and their production and use.

(57) Diphenylalkanoamine derivatives of the formula:

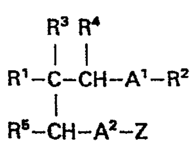

wherein

$R^1$ and $R^2$ are each independently an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio;

$R^3$, $R^4$ and $R^5$ are represented by one of the following groups: (a) $R^3$ = OH, $R^4$ = $R^5$ = H; (b) $R^3$ = $R^4$ = $R^5$ = H; (c) $R^4$ = H, $R^3$ and $R^5$ together indicate a covalent bond; (d) $R^5$ = H, $R^3$ and $R^4$ together indicate a covalent bond;

Z is a group of the formula:

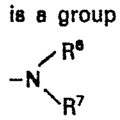

(wherein $R^6$ and $R^7$ are each independently a hydrogen atom or a $C_1$-$C_4$ alkyl group or, when taken together with the adjacent nitrogen atom to which they are attached, represent piperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino);

$A^1$ is a $C_1$-$C_5$ alkylene group; and

$A^2$ is a $C_2$-$C_5$ alkylene group,

and non-toxic, pharmaceutically acceptable acid addition salts thereof, which have cerebral vasodilating activity and antihypoxic activity.

EP 0 080 721 A2

DIPHENYLALKANOAMINE DERIVATIVES, AND THEIR PRODUCTION AND USE

This invention relates to diphenylalkanoamine derivatives having useful pharmacological activities such as cerebral vasodilating activity and antihypoxic activity, and their production and use.

The said diphenylalkanoamine derivatives are represented by the formula:

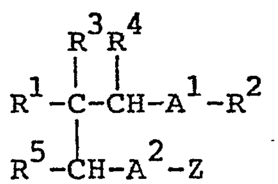

(I)

wherein

$R^1$ and $R^2$ are each independently an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio;

$R^3$, $R^4$ and $R^5$ are represented by one of the following groups: (a) $R^3$ = OH, $R^4$ = $R^5$ = H; (b) $R^3$ = $R^4$ = $R^5$ = H; (c) $R^4$ = H, $R^3$ and $R^5$ together indicate a covalent bond; (d) $R^5$ = H, $R^3$ and $R^4$ together indicate a covalent bond;

Z is a group of the formula:

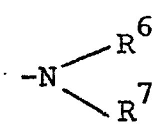

(wherein $R^6$ and $R^7$ are each independently a hydrogen atom or a $C_1$-$C_4$ alkyl group or, when taken together with the

adjacent nitrogen atom to which they are attached, represent piperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino);

$A^1$ is a $C_1$-$C_5$ alkylene group; and

$A^2$ is a $C_2$-$C_5$ alkylene group.

In the significances as defined above, the term "halogen" includes fluorine, chlorine, bromine and iodine; the term "ar($C_1$-$C_4$)alkyl" covers benzyl, phenylethyl, phenylisopropyl, etc.; and the term "aryl" means phenyl, pyridyl, thienyl, furyl, naphthyl or the like. The terms "$C_1$-$C_4$ alkyl" and "$C_1$-$C_4$ alkoxy" mean respectively straight or branched chain alkyl and alkoxy groups having from 1 to 4 carbon atoms (e.g. methyl, ethyl, n-propyl, isopropyl, n-butyl, methoxy, ethoxy, n-propoxy, isopropoxy, n-butoxy); and the terms "$C_1$-$C_5$ alkylene" and "$C_2$-$C_5$ alkylene" mean straight or branched chain alkylene groups (e.g. methylene, ethylene, trimethylene, tetramethylene, pentamethylene, methylbutylene).

It has now been unexpectedly found that the diphenylalkanoamine derivatives (I) have various excellent pharmacological activities, particularly cerebral and coronary vasodilating activity, antihypoxic activity, antispasmodic activity and inhibitory action of aggregation of blood platelets.

Accordingly, the diphenylalkanoamine derivatives

(I) are therapeutically useful to treat cerebral or coronary arteriosclerosis, senile mental indolence and the results of cerebral insufficiency. From this point of view, preferred are the compounds of the formula (I) wherein $R^1$ is phenyl or substituted phenyl, $R^2$ is phenyl and $A^1$ is ethylene or trimethylene.

A basic object of the present invention is to provide novel diphenylalkanoamine derivatives (I) having cerebral vasodilating activity, antihypoxic activity and the like. Another object of the invention is to provide a process for producing the diphenylalkanoamine derivatives (I). These and other objects will be apparent to those skilled in the art to which the present invention pertains from the foregoing and subsequent descriptions.

According to the present invention, the diphenylalkanoamine derivatives (Ia) of the formula:

$$\begin{array}{l} \quad\ \ OH \\ \quad\ \ | \\ R^1\text{-}C\text{-}CH\text{-}A^1\text{-}R^2 \\ \quad\ \ | \\ \quad\ \ CH_2\text{-}A^2\text{-}Z \end{array} \qquad (Ia)$$

wherein $R^1$, $R^2$, $A^1$, $A^2$ and Z are each as defined above can be prepared by reacting a compound of the formula:

$$\begin{array}{l} R^1\text{-}C\text{-}CH_2\text{-}A^1\text{-}R^2 \\ \quad\ \ \| \\ \quad\ \ O \end{array} \qquad (II)$$

wherein $R^1$, $R^2$ and $A^1$ are each as defined above with a Grignard reagent of the formula:

$$X\text{-}Mg\text{-}CH_2\text{-}A^2\text{-}Z' \qquad (III)$$

wherein X is a chlorine atom or a bromine atom, Z' is a

group of the formula:

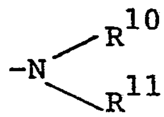

(wherein $R^{10}$ and $R^{11}$ are each independently a benzyl group or a $C_1$-$C_4$ alkyl group or, when taken together with the adjacent nitrogen atom to which they are attached, represent benzylpiperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-aryl-piperazino or N-ar($C_1$-$C_4$)alkylpiperazino) and $A^2$ is as defined above and optionally subjecting the resulting product to benzylation of the hydroxyl group and/or debenzylation of the benzyloxy, benzylamino, dibenzylamino or benzylpiperazino group.

The diphenylalkanoamine derivatives (Ib) of the formula:

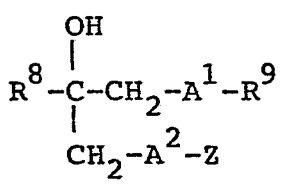

(Ib)

wherein $A^1$, $A^2$ and Z are each as defined above and $R^8$ and $R^9$ are each independently an aryl group optionally substituted with 1 to 2 substituents selected from the group consisting of halogen, $C_1$-$C_4$ alkyl, benzyloxy, $C_1$-$C_4$ alkoxy, di($C_1$-$C_4$)-alkylamino and $C_1$-$C_4$ alkylthio can be prepared by reacting a compound of the formula:

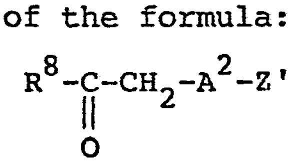

(IV)

wherein $R^8$, $A^2$ and Z' are each as defined above with a

Grignard reagent of the formula:

$$X\text{-}Mg\text{-}CH_2\text{-}A^1\text{-}R^9 \qquad (V)$$

wherein X, $A^1$ and $R^9$ are each as defined above and optionally subjecting the resulting product to debenzylation of the benzylamino, dibenzylamino or benzylpiperazino group.

The diphenylalkanoamine derivatives (Ia) can be prepared as well by reacting the compound (II) with a compound of the formula:

$$LiCH_2\text{-}A^2\text{-}Z' \qquad (VI)$$

wherein $A^2$ and Z' are each as defined above and optionally subjecting the resulting product to benzylation of the hydroxyl group and/or debenzylation of the benzyloxy, benzylamino, dibenzylamino or benzylpiperazino group.

The diphenylalkanoamine derivatives (Ib) can be prepared as well by reacting a compound of the formula (IV) with a compound of the formula:

$$LiCH_2\text{-}A^1\text{-}R^9 \qquad (VII)$$

wherein $A^1$ and $R^9$ are each as defined above and optionally subjecting the resulting product to debenzylation of the benzylamino, dibenzylamino or benzylpiperazino group.

A mixture of the diphenylalkanoamine derivatives (Ic) and (Id) of the formulas:

$$R^1\text{-}\underset{\underset{CH\text{-}A^2\text{-}Z}{\|}}{C}\text{-}CH_2\text{-}A^1\text{-}R^2 \qquad (Ic)$$

$$R^1\text{-}\underset{\underset{CH_2\text{-}A^2\text{-}Z}{|}}{C}\text{=}CH\text{-}A^1\text{-}R^2 \qquad (Id)$$

wherein $R^1$, $R^2$, $A^1$, $A^2$ and Z are each as defined above can be prepared by dehydrating the compound (Ia).

The diphenylalkanoamine derivatives (Ie) of the formula:

$$\begin{array}{l} R^1\text{-}CH\text{-}CH_2\text{-}A^1\text{-}R^2 \\ \quad\ \ | \\ \quad\ CH_2\text{-}A^2\text{-}Z \end{array} \qquad \text{(Ie)}$$

wherein $R^1$, $R^2$, $A^1$, $A^2$ and Z are each as defined above can be prepared by reducing a mixture or each of the compounds (Ic) and (Id) and optionally subjecting the resulting product to benzylation of the hydroxyl group and/or debenzylation of the benzyloxy or benzylpiperazino group.

The diphenylalkanoamine derivatives (Ic) can be prepared by reducing a compound of the formula:

$$\begin{array}{l} R^1\text{-}C\text{-}CH_2\text{-}A^1\text{-}R^2 \\ \quad\ \| \\ \quad\ CH\text{-}A^3\text{-}COZ \end{array} \qquad \text{(VIII)}$$

wherein $R^1$, $R^2$, $A^1$ and Z are each as defined above and $A^3$ is a $C_1$-$C_4$ alkylene group and optionally subjecting the resulting product to demethylation of the N-alkyl-N-methylamino, dimethylamino or N-methylpiperazino group.

The compound (VIII) can be prepared by reacting a compound of the formula:

$$\begin{array}{l} R^1\text{-}C\text{-}CH_2\text{-}A^1\text{-}R^2 \\ \quad\ \| \\ \quad\ CH\text{-}A^3\text{-}COOH \end{array} \qquad \text{(IX)}$$

wherein $R^1$, $R^2$, $A^1$ and $A^3$ are each as defined above with a compound of the formula:

$$HZ \qquad \text{(X)}$$

wherein Z is as defined above.

The compound (IX) can be prepared by reacting a

compound (II) with a Wittig reagent of the formula:

$$(C_6H_5)_3P{=}CH{-}A^3{-}COOH \qquad \text{(XI)}$$

wherein $A^3$ is as defined above.

The diphenylalkanoamine derivatives (Id) can be prepared by reacting a compound of the formula:

$$R^1{-}\underset{\underset{O}{\|}}{C}{-}CH_2{-}A^2{-}Z \qquad \text{(XII)}$$

wherein $R^1$, $A^2$ and Z are each as defined above with a Wittig reagent of the formula:

$$(C_6H_5)_3P{=}CH{-}A^1{-}R^2 \qquad \text{(XIII)}$$

wherein $R^2$ and $A^1$ are each as defined above and optionally subjecting the resulting product to demethylation of the N-alkyl-N-methylamino, dimethylamino or N-methylpiperazino group.

Th preparation processes as stated above will be illustrated below in detail.

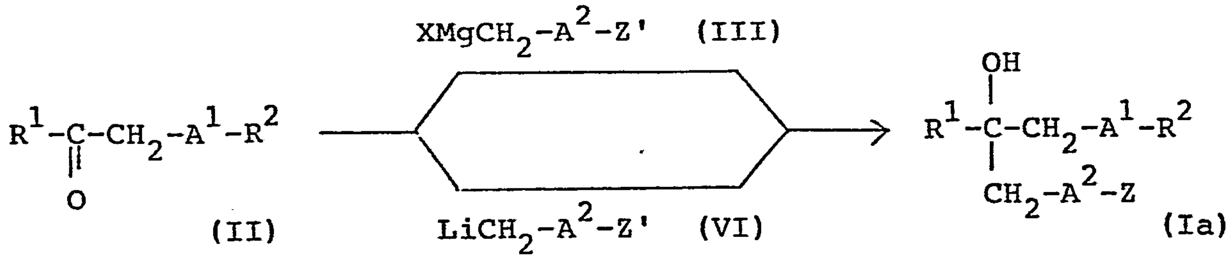

(III) (II) (VI) (Ia)

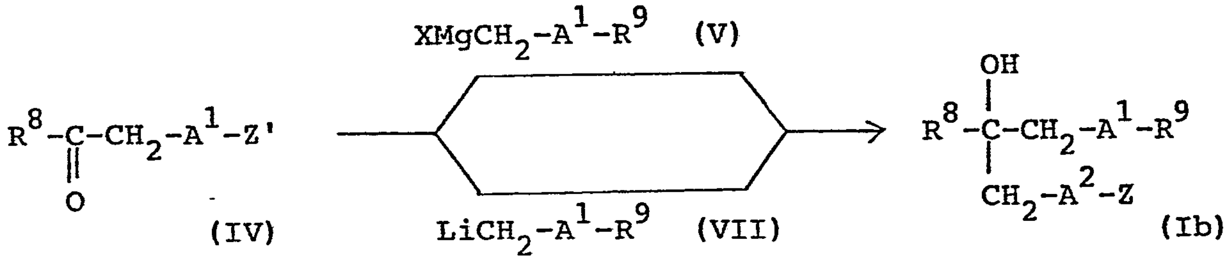

(V) (IV) (VII) (Ib)

(Ia)

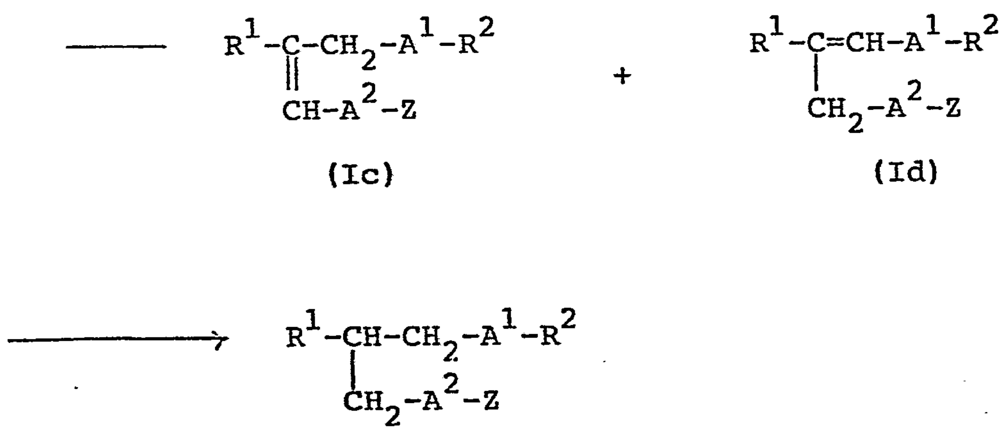

(Ic) + (Id)

(Ie)

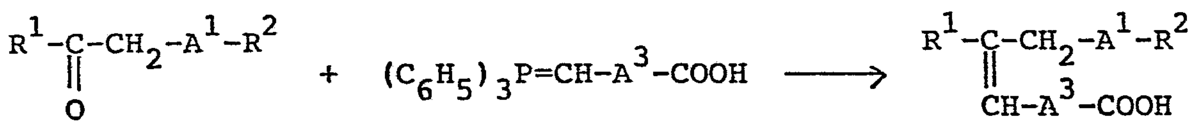

(II) + (XI) (IX)

(IX) + HZ

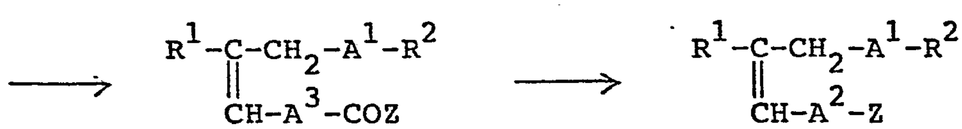

(X) (VIII) (Ic)

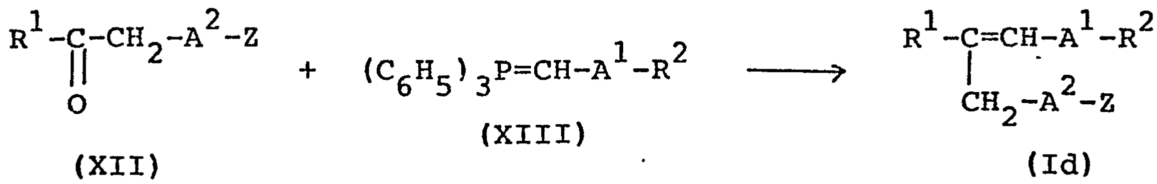

(XII) (XIII) (Id)

1. Production of the compound (Ia) from the compouds (II) and (III):-

The compound (II) is reacted with the compound (III) usually in an inert solvent (e.g. ether, tetrahydrofuran) at a temperature ranging from 0°C to the refluxing temperature of the reaction system, optionally followed by subjecting the resulting product to benzylation of the hydroxyl group and/or debenzylation of the benzyloxy, benzylamino, dibenzylamino or benzylpiperazino group to give the compound (Ia).

2. Production of the compound (Ib) from the compounds (IV) and (V):-

The compound (IV) is reacted with the compound (V) usually in an inert solvent (e.g. ether, tetrahydrofuran) at a temperature ranging from 0°C to the refluxing temperature of the reaction system, optionally followed by subjecting the resulting product to debenzylation of the benzylamino, dibenzylamino or benzylpiperazino group to give the compound (Ib).

3. Production of the compound (Ia) from the compounds (II) and (VI):-

The compound (II) is reacted with the compound (VI) usually in an inert solvent (e.g. ether, tetrahydrofuran) at a temperature ranging from -70°C to 20°C, optionally followed by subjecting the resulting product to benzylation of the hydroxyl group and/or debenzylation of the benzyloxy, benzylamino, dibenzylamino or benzylpiperazino group to give the compound (Ia).

4. Production of the compound (Ib) from the compounds (IV) and (VII):-

The compound (IV) is reacted with the compound (VII) usually in an inert solvent (e.g. ether, tetrahydrofuran) at a temperature ranging from -70°C to 20°C, optionally followed by subjecting the resulting product to debenzylation of the benzylamino, dibenzylamino or benzylpiperazino group to give the compound (Ib).

5. Production of a mixture of the compounds (Ic) and (Id) from the compound (Ia):-

5-1. The compound (Ia) is reacted with an acid (e.g. hydrochloric acid, hydrobromic acid, sulfuric acid, phosphorus tribromide) in an inert solvent (e.g. methanol, ethanol, isopropanol, water) at a temperature ranging from 30°C to the refluxing temperature of the reaction system to give a mixture of the compounds (Ic) and (Id).

5-2. The compound (Ia) is reacted with a dehydrating reagent (e.g. thionyl chloride, phosphorus oxychloride) in pyridine at a temperature ranging from -20°C to 20°C to give a mixture of the compounds (Ic) and (Id).

6. Production of the compound (Ie) from a mixture or each of the compounds (Ic) and (Id):-

6-1. The reduction may be accomplished by catalytic hydrogenation using a catalyst (e.g. palladium on charcoal, platinum oxide, Rany nickel) in an inert solvent (e.g. methanol, ethanol/water, isopropanol, dioxane) at a temperature ranging from 0°C to 50°C, optionally followed by subjecting the resulting product to benzylation of hydroxyl group and/or debenzylation of the benzyloxy or benzylpiperazino group to give the compound (Ie).

6-2. The reduction may be accomplished by diimide reduction in a solvent (e.g. methanol, ethanol, water, acetic acid) at a temperature ranging from -30°C to 30°C, optionally followed by subjecting the resulting product to benzylation of the hydroxyl group and/or debenzylation of the benzyloxy or benzylpiperazino group to give the compound (Ie).

7. Production of the compound (IX) from the compounds (II) and (XI):-

The method for the Wittig reaction is described in Organic Reactions, 14, Capt., 3 (1965), John Wiley & Sons, Inc. Preferably, the Wittig reaction is carried out in an inert organic solvent such as dimethylsulfoxide, usually at a temperature from 10°C to 60°C.

The phosphorane of the formula (XI) can be obtained by reaction of dimusyl sodium or potassium t-butoxide with (ω-carboxyalkyl)triphenylphosphonium halide.

8. Production of the compound (VIII) from the compounds (IX) and (X):-

8-1. The compounds (IX) and (X) are reacted together with dicyclohexylcarbodiimide in an inert solvent (e.g. dichloromethane, chloroform, tetrahydrofuran) at a temperature ranging from 10°C to the refluxing temperature of the reaction system to give the compound (VIII).

8-2. Alkyl chloroformate (e.g. ethyl chloroformate, isobutyl chloroformate) was added to a mixture of the compound (IX) and a tertiary amine (triethylamine, N-methylmorphorine) in an inert solvent at a temperature ranging from -20°C to 10°C, followed by addition of the compound (X) to give the compound (VIII).

9. Production of the compound (Ic) from the compound (VIII):-

The reaction may be accomplished by a known reducing agent for the conversion of an amido group to an amino group (e.g. lithium aluminum hydride, lithium aluminum trimethoxy hydride) in an inert solvent (e.g. ether, tetrahydrofuran) at a temperature ranging from 30°C to the refluxing temperature of the reaction system, optionally followed by subjecting the resulting product to demethylation of the N-alkyl-N-methylamino, dimethylamino or N-methylpiperazino group.

10. Production of the compound (Id) from the compounds (XII) and (XIII):-

The method for the Wittig reaction is described in Organic Reactions, 14, Capt., 3 (1965), John Wiley & Sons, Inc. Preferably, the Wittig reaction is carried out in an

inert solvent such as tetrahydrofuran or dimethylsulfoxide, usually at a temperature from 10°C to 60°C, optionally followed by subjecting the resulting product to demethylation of the N-alkyl-N-methylamino, N-methylpiperazino or dimethylamino group.

The phosphorane of the formula (XIII) can be obtained by reaction of dimusyl sodium or potassium t-butoxide with aralkyltriphenylphosphonium halide.

11. Debenzylation of the benzyloxy, benzylamino, dibenzylamino or benzylpiperazino group:-

The reaction may be accomplished by a per se conventional reduction procedure such as catalytic hydrogenation using palldium on charcoal as the catalyst.

12. Benzylation of the hydroxyl group:-

The reaction may be accomplished by a per se conventional procedure, preferably by treatment with benzyl chloride in the presence of an alkali.

13. Demethylation of the dimethylamino, N-($C_1$-$C_4$)alkyl-N-methylamino or N-methylpiperazino group:-

This reaction may be accomplished by a per se conventional procedure, preferably by treatment with ethyl chloroformate, followed by hydrolysis with an alkali.

Specific examples of the diphenylalkanoamine derivatives (I) are as follows:

1-[3-(N,N-Dimethylamino)propyl]-1,4-diphenyl-1-butanol;

1-[3-(N,N-Dimethylamino)propyl]-1,5-diphenyl-1-pentanol;

1-(3-Piperidinopropyl)-1-(p-fluorophenyl)-4-phenyl-1-butanol;

1-[3-(N,N-Dimethylamino)propyl]-1,4-diphenylbutane;

1-[3-(N,N-Dimethylamino)propyl]-1,5-diphenylpentane;

1-(3-Piperidinopropyl)-1-(p-fluorophenyl)-4-phenylbutane;

1-[3-(N,N-Dimethylamino)propyl]-1,5-diphenyl-1-pentene;

1-(3-Piperidinopropyl)-1-(p-fluorophenyl)-4-phenyl-1-butene;

N,N-Dimethyl-N-(4,8-diphenyl-3-octen-1-yl)amine;

N-[4-(p-Fluorophenyl)-7-phenyl-3-hepten-1-yl]-piperidine;

N-(6,9-Diphenyl-5-nonen-1-yl)piperidine:

1-(3-Piperidinopropyl)-1-(o-chlorophenyl)-5-phenylpentane;

1-(3-Pyrrolidinopropyl)-1-(p-ethylphenyl)-4-phenylbutane;

1-[3-(N,N-Dimethylamino)propyl]-1-(p-methoxyphenyl)-4-phenylbutane;

1-(3-Hexamethyleneiminopropyl)-1,4-diphenylbutane;

1-[3-(N-Methylpiperazino)propyl)]-1,4-diphenyl-1-butene;

1-[3-(4-Phenylpiperidino)propyl]-1,4-diphenyl-1-

butene;

1-(5-Piperidinopentyl)-1,4-diphenylbutane;

N-(6,9-Diphenyl-5-nonen-1-yl)morpholine;

1-(5-Morpholinopentyl)-1,4-diphenylbutane;

1-(3-Morpholinopropyl)-1,5-diphenyl-1-pentanol;

1-(3-Morpholinopropyl)-1,5-diphenyl-1-pentene;

N-(4,8-Diphenyl-3-octen-1-yl)morpholine;

1-(3-Morpholinopropyl)-1,5-diphenylpentane;

1-(3-Pyrrolidinopropyl)-1,6-diphenylhexane;

1-(3-Piperidinopropyl)-1-(2-thienyl)-5-phenylpentane;

1-(3-(Morpholinopropyl)-1-(2-furyl)-3-phenylpropane;

1-(4-Morpholinobutyl)-1-(2-pyridyl)-5-phenyl-1-pentene;

1-(3-Morpholinopropyl)-1-(4-pyridyl)-4-phenyl-1-butene;

1-(3-Piperidinopropyl)-1-(4-hydroxyphenyl)-4-phenylbutane;

1-(3-Piperidinopropyl)-1-(3-aminophenyl)-4-phenylbutane;

1-(3-Morpholinopropyl)-1-(4-dimethylaminophenyl)-4-phenylbutane;

1-(3-Morpholinopropyl)-1-(4-ethylthiophenyl)-4-phenylbutane;

1-(3-Morpholinopropyl)-1-(4-benzyloxyphenyl)-4-phenylbutane;

1-(3-Methylaminopropyl)-1,4-diphenylbutane;

1-(3-Aminopropyl)-1,4-diphenylbutane;

1-(3-Piperazinopropyl)-1,4-diphenylbutane;

1-(3-Piperidinopropyl)-1,7-diphenylheptane;

1-(6-Morpholinohexyl)-1,4-diphenylpentane;

1-[3-(4-Methylpiperidino)propyl]-1,4-diphenylbutane;

1-[3-(4-Benzylpiperidino)propyl]-1,5-diphenylpentane;

1-[3-(4-Phenylpiperazino)propyl]-1-(β-naphthyl)-4-phenylbutane;

1-[3-(4-Benzylpiperazino)propyl]-1-(o-bromophenyl)-4-phenylpentane, etc.

The thus prepared diphenylalkanoamine derivatives (I) can be readily converted into their organic or inorganic acid addition salts by a conventional procedure.

For preparation of the pharmaceutical composition, the diphenylalkanoamine derivatives (I) or their salts may be admixed with carriers, diluents, lubricants, fillers and/or binders (e.g. lactose, sucrose, calcium phosphate, starch, talcum, casein, magnesium stearate, methyl cellulose, polyglycols, tragacanth). When desired, there may be also incorporated stabilizers and/or emulsifying agents. The resulting mixture may be processed in a usual manner to make tablets, capsules, pills, ampoules and the like. The usual oral dosage of the active ingredient may be from about 10 to 200 mg per day.

Practical and presently preferred embodiments of the invention are illustratively shown in the following Examples without limiting the scope of the invention in any way.

Example 1

A solution of 7 g of γ-dimethylaminopropyl chloride in 20 ml of dry ether was added dropwise to a mixture of 0.6 g of magnesium and 15 ml of dry ether in 30 minutes, and the mixture was heated under refluxing for 30 minutes. After cooling, a solution of 5 g of 1,4-diphenyl-1-butanone in 10 ml of tetrahydrofuran was added dropwise thereto, and the mixture was stirred for additional 2 hours. The reaction mixture was poured into dilute hydrochloric acid, followed by adding ammonium water thereto to adjust a pH of 10. The resultant mixture was extracted with ethyl acetate. The extract was washed with water, dried over anhydrous sodium sulfate and concentrated under reduced pressure. The residue was purified by column chromatography on silica gel to give 1-[3-(N,N-dimethylamino)propyl]-1,4-diphenyl-1-butanol. M.P., 92 - 93.5°C (oxalate).

Example 2

In a similar manner to Example 1, the following compounds were prepared:

1-(3-Piperidinopropyl)-1-(p-fluorophenyl)-4-phenyl-1-butanol. NMR ($CDCl_3$) δ ppm: 1.0 - 2.0 (14H, m), 2.53 (2H, t), 2.0 - 2.7 (7H, m), 6.8 - 7.5 (9H, m);

1-[3-(N,N-Dimethylamino)propyl]-1,5-diphenyl-1-

pentanol. NMR ($CDCl_3$) δ ppm: 0.8 - 1.9 (10H, m), 2.07 (6H, s), 2.1 - 2.6 (4H, m), 6.95 - 7.5 (10H, m).

Example 3

(1) A mixture of 2.5 g of 1-[3-(N,N-dimethylamino)propyl]-1,4-diphenyl-1-butanol, 120 ml of ethanol and 3 ml of hydrochloric acid was heated under refluxing for 4 hours. After cooling, the reaction mixture was poured into dilute ammonium water and extracted with ethyl acetate. The extract was washed with water, dried over anhydrous sodium sulfate and concentrated under reduced pressure. The residue was purified by column chromatography on silica gel to give a mixture of 1-[3-(N,N-dimethylamino)propyl]-1,4-diphenyl-1-butene and N,N-dimethyl-N-(4,7-diphenyl-3-hepten-1-yl)amine. NMR ($CDCl_3$) δ ppm: 1.20 - 2.83 (16H, m), 5.30 - 5.80 (1H, m), 6.80 - 7.40 (10H, m).

(2) The above obtained mixture (0.7 g) was added to a suspension of 0.2 g of 10 % palladium on charcoal in 0.5 ml of water and 20 ml of ethanol, and the resultant mixture was stirred in hydrogen atmosphere at room temperature for 30 minutes. After the catalyst was filtered off, the filtrate was poured into water and extracted with ethyl acetate. The extract was dried over anhydrous sodium sulfate and concentrated under reduced pressure to give 1-[3-(N,N-dimethylamino)propyl]-1,4-diphenylbutane. M.P., 97 - 98°C (oxalate).

Example 4

(1) In a similar manner to Example 3 (1), the

following mixtures were prepared:

A mixture of 1-(3-piperidinopropyl)-1-(p-fluorophenyl)-4-phenyl-1-butene and N-[4-(p-fluorophenyl)-7-phenyl-3-hepten-1-yl]piperidine, pale yellow oil. NMR ($CDCl_3$) δ ppm: 1.2 - 1.85 (8H, m), 1.85 - 2.9 (12H, m), 5.33 - 5.70 (1H, m), 6.80 - 7.40 (9H, m);

A mixture of 1-[3-(N,N-dimethylamino)propyl]-1,5-diphenyl-1-pentene and N,N-dimethyl-N-(4,8-diphenyl-3-octen-1-yl)amine, pale yellow oil. NMR ($CDCl_3$) δ ppm: 1.20 - 2.80 (18H, m), 5.23 - 5.80 (1H, m), 6.80 - 7.40 (10H, m).

(2) In a similar manner to Example 3 (2), the following compounds were prepared:

1-(3-Piperidinopropyl)-1-(p-fluorophenyl)-4-phenylbutane, M.P., 128 - 129°C (oxalate);

1-[3-(N,N-Dimethylamino)propyl]-1,5-diphenylpentane, M.P., 135 - 136°C (oxalate).

Example 5

(1) A mixture of 3.42 g of sodium hydride (content, 60 % w/w) and 60 ml of dimethyl sulfoxide was stirred at 65 - 70°C for 1 hour. The solution thus obtained was added dropwise to 18.4 g of (4-carboxybutyl)triphenylphosphonium bromide, and the resultant mixture was cooled with an ice-bath. To the mixture was added dropwise a solution of 6 g of 1,4-diphenyl-1-butanone in 30 ml of dimethyl sulfoxide, and the resulting mixture was stirred at 20 - 25°C for 15 hours. The reaction mixture was combined with 1 liter of ice-water, acidified to pH 4 with an aqueous

solution of potassium bisulfate and extracted with ether. The extract was washed with water and a saturated aqueous solution of sodium chloride, dried over anhydrous magnesium sulfate and concentrated under reduced pressure. The residue was purified by column chromatography on silica gel to give 6,9-diphenylnon-5-enoic acid having the following structure:

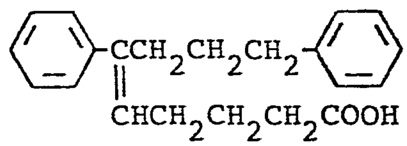

NMR ($CDCl_3$) δ ppm: 1.40 - 2.75 (18H, m), 5.38, (1.2H, t), 5.59 (0.8H, t), 6.90 - 7.33 (10H, m).

(2) To a solution of 2.9 g of 6,9-diphenylnon-5-enoic acid and 1.01 g of triethylamine in 50 ml of tetrahydrofuran was added a solution of 1 g of chloroorthoformate in 5 ml of tetrahydrofuran at -10°C - 0°C. After stirring for 30 minutes at -10°C - 0°C, a solution of 1.6 g of piperidine in 5 ml of tetrahydrofuran was added thereto, and the mixture was stirred for additional 1 hour. The reaction mixture was poured into water and extracted with ethyl acetate. The extract was washed with dilute aqueous hydrochloric acid, water and a saturated aqueous sodium hydrogencarbonate in order, dried over anhydrous sodium sulfate, concentrated under reduced pressure and purified by column chromatography on silica gel to give N-(6,9-diphenylnon-5-enoyl)piperidine, brownish oil. NMR ($CDCl_3$) δ ppm: 3.2 - 3.65 (4H, m), 5.46 (1H, t), 7.0 - 7.9 (10H, m).

(3) To a mixture of 0.35 g of lithium aluminum

hydride and 20 ml of tetrahydrofuran was added a solution of 1.5 g of N-(6,9-diphenylnon-5-enoyl)piperidine at room temperature. After refluxing for 1 hour, water was added to the reaction mixture. The resulting mixture was poured into water and extracted with ethyl acetate. The extract was washed with water, dried over anhydrous sodium sulfate, concentrated under reduced pressure and purified by column chromatography on silica gel to give N-(6,9-diphenyl-5-nonenyl)piperidine having the following structure as a pale yellow oil:

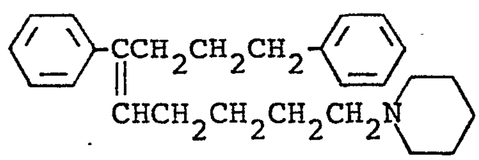

NMR ($CDCl_3$) δ ppm: 1.2 - 2.7 (24H, m), 5.41 (0.7H, t), 5.61 (0.3H, t), 6.9 - 7.6 (10H, m).

Example 6

To a solution of 4.61 g of 3-phenylpropyltriphenylphosphonium bromide in 40 ml of tetrahydrofuran was added 1.12 g of potassium t-butoxide at room temperature. After stirring at room temperature for 15 minutes, a solution of 1 g of γ-morpholino-p-fluorobutyrophenone in 10 ml of tetrahydrofuran was added, and the mixture was stirred for additional 1 hour. The reaction mixture was poured into water and extracted with ethyl acetate. The extract was washed with water, dried over anhydrous sodium sulfate, concentrated under reduced pressure and purified by column chromatography on silica gel to give N-[4-(p-fluorophenyl)-7-phenyl-4-hepten-1-yl]morpholine, brownish oil. NMR ($CDCl_3$) δ ppm: 3.5 - 3.85 (4H, t-like), 5.47 (1H, t), 6.8 - 7.6 (9H, m).

Example 7

A solution of 2.18 g of 1-bromo-3-phenylpropane in 10 ml of dry ether was added dropwise to a mixture of 0.264 g of magnesium and 20 ml of dry ether in 30 minutes, and the mixture was heated under refluxing for 30 minutes. After cooling, a solution of 2.51 g of γ-morpholino-p-fluorobutyrophenone in 20 ml of tetrahydrofuran was added dropwise, and the mixture was stirred for additional 2 hours. The reaction mixture was poured into dilute hydrochloric acid, followed by addition of ammonium water to make pH 10. The resulting mixture was extracted with ethyl acetate. The extract was washed with water, dried over anhydrous sodium sulfate and concentrated under reduced pressure. The residue was purified by column chromatography on silica gel to give 1-(3-morpholinopropyl)-1-(4-fluorophenyl)-4-phenyl-1-butanol, pale yellow oil. NMR ($CDCl_3$) δ ppm: 1.1 - 2.0 (8H, m), 2.0 - 2.7 (9H, m), 3.68 (4H, t), 6.8 - 7.5 (9H, m).

What is claimed is:

1. A compound of the formula:

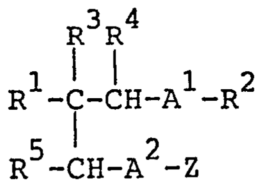

wherein

$R^1$ and $R^2$ are each independently an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio;

$R^3$, $R^4$ and $R^5$ are represented by one of the following groups: (a) $R^3$ = OH, $R^4$ = $R^5$ = H; (b) $R^3$ = $R^4$ = $R^5$ = H; (c) $R^4$ = H, $R^3$ and $R^5$ together indicate a covalent bond; (d) $R^5$ = H, $R^3$ and $R^4$ together indicate a covalent bond;

Z is a group of the formula:

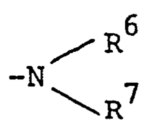

(wherein $R^6$ and $R^7$ are each independently a hydrogen atom or a $C_1$-$C_4$ alkyl group or, when taken together with the adjacent nitrogen atom to which they are attached, represent piperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino);

$A^1$ is a $C_1$-$C_5$ alkylene group; and

$A^2$ is a $C_2$-$C_5$ alkylene group, or a non-toxic, pharmaceutically acceptable acid addition salt thereof.

2. The compound according to claim 1, wherein $R^1$ and $R^2$ are each independently a phenyl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio.

3. The compound according to claim 1, wherein $R^1$ is a thienyl group.

4. The compound according to claim 1, wherein $R^1$ is a pyridyl group.

5. The compound according to claim 1, wherein $R^1$ is a furyl group.

6. The compound according to claim 1, wherein Z is a morpholino group.

7. The compound according to claim 1, wherein Z is a dialkylamino group.

8. The compound according to claim 1, wherein Z is a piperidino group.

9. The compound according to claim 1, wherein Z is a pyrrolidino group or a hexamethyleneimino group.

10. The compound according to claim 1, wherein $A^1$ is an ethylene group or a trimethylene group.

11. The compound of the formula:

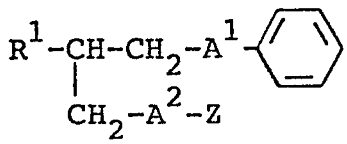

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio, $A^1$ and $A^2$ are each as defined in claim 1 and Z is a morpholino group.

12. The compound of the formula:

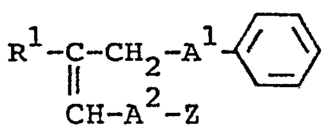

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio, $A^1$ and $A^2$ are each as defined in claim 1 and Z is a morpholino group.

13. The compound of the formula:

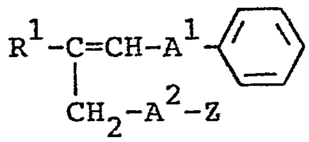

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio, $A^1$ and $A^2$ are each as defined in claim 1 and Z is a morpholino group.

14. The compound of the formula:

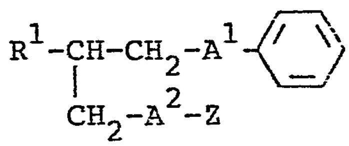

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio, $A^1$ and $A^2$ are each as defined in claim 1 and Z is a piperidino group.

15. The compound of the formula:

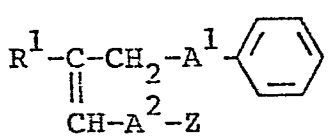

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy,

amino, $C_1$-$C_4$ alkylamino, di($C_1$-$C_4$)alkylamino and $C_1$-$C_4$ alkylthio, $A^1$ and $A^2$ are each as defined in claim 1 and Z is a piperidino group.

16. The compound of the formula:

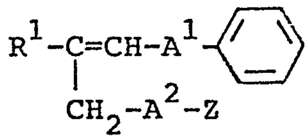

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio, $A^1$ and $A^2$ are each as defined in claim 1 and Z is a piperidino group.

17. The compound of the formula:

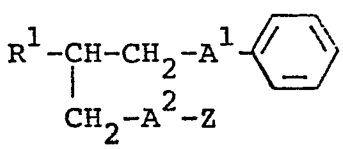

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio, $A^1$ and $A^2$ are each as defined in claim 1 and Z is a di($C_1$-$C_4$)alkylamino group.

18. The compound of the formula:

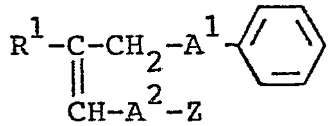

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di$(C_1$-$C_4)$alkylamino and $C_1$-$C_4$ alkylthio, $A^1$ and $A^2$ are each as defined in claim 1 and Z is a di$(C_1$-$C_4)$alkylamino group.

19. The compound of the formula:

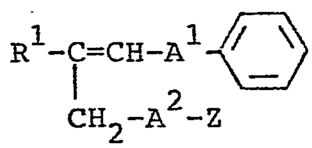

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di$(C_1$-$C_4)$alkylamino and $C_1$-$C_4$ alkylthio, $A^1$ and $A^2$ are each as defined in claim 1 and Z is a di$(C_1$-$C_4)$alkylamino group.

20. The compound of the formula:

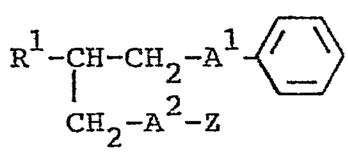

wherein $R^1$ is an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di$(C_1$-$C_4)$alkylamino and $C_1$-$C_4$

alkylthio, $A^1$ and $A^2$ are each as defined in claim 1 and Z is a piperazino group, an N-$(C_1-C_4)$alkylpiperazino group, an N-arylpiperazino group, an N-ar$(C_1-C_4)$alkylpiperazino group, a $C_1-C_4$ alkylpiperidino group, an arylpiperidino group or an ar$(C_1-C_4)$alkylpiperidino group.

21. The compound of the formula:

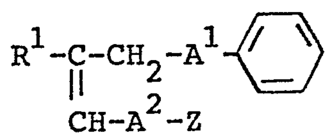

wherein $R^1$ is an aryl group optionally substituted with 1 to 2 substituents selected from the group consisting of halogen, $C_1-C_4$ alkyl, benzyloxy, hydroxyl, $C_1-C_4$ alkoxy, amino, $C_1-C_4$ alkylamino, di$(C_1-C_4)$alkylamino and $C_1-C_4$ alkylthio, $A^1$ and $A^2$ are each as defined in claim 1 and Z is a piperazino group, an N-$(C_1-C_4)$alkylpiperazino group, an N-arylpiperazino group, an N-ar$(C_1-C_4)$alkylpiperazino group, a $C_1-C_4$ alkylpiperidino group, an arylpiperidino group or an ar$(C_1-C_4)$alkylpiperidino group.

22. The compound of the formula:

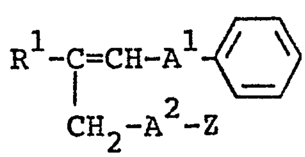

wherein $R^1$ is an aryl group optionally substituted with 1 to 2 substituents selected from the group consisting of halogen, $C_1-C_4$ alkyl, benzyloxy, hydroxyl, $C_1-C_4$ alkoxy, amino, $C_1-C_4$ alkylamino, di$(C_1-C_4)$alkylamino and $C_1-C_4$

alkylthio, $A^1$ and $A^2$ are each as defined in claim 1 and Z is a piperazino group, an N-$(C_1-C_4)$alkylpiperazino group, an N-arylpiperazino group, an N-ar$(C_1-C_4)$alkylpiperazino group, a $C_1-C_4$ alkylpiperidino group, an arylpiperidino group or an ar$(C_1-C_4)$alkylpiperidino group.

23. The compound of the formula:

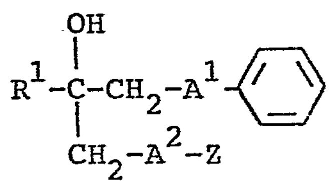

wherein $R^1$ is an aryl group optionally substituted with 1 to 2 substituents selected from the group consisting of halogen, $C_1-C_4$ alkyl, benzyloxy, hydroxyl, $C_1-C_4$ alkoxy, amino, $C_1-C_4$ alkylamino, di$(C_1-C_4)$alkylamino and $C_1-C_4$ alkylthio, $A^1$ and $A^2$ are each an ethylene group or a trimethylene group and Z is a piperidino group, a morpholino group or a di$(C_1-C_4)$alkylamino group.

24. 1-[3-(N,N-Dimethylamino)propyl]-1,4-diphenyl-1-butanol.

25. 1-(3-Piperidinopropyl)-1-(p-fluorophenyl)-4-phenyl-1-butanol.

26. 1-[3-(N,N-Dimethylamino)propyl]-1,5-diphenyl-1-pentanol.

27. 1-[3-(N,N-Dimethylamino)propyl]-1,4-diphenylbutane.

28. 1-(3-Piperidinopropyl)-1-(p-fluorophenyl)-4-phenylbutane.

29. 1-[3-(N,N-Dimethylamino)propyl]-1,5-diphenylpentane.

30. N-[6,9-Diphenyl-5-nonen-1-yl)piperidine.

31. N-[4-(p-Fluorophenyl)-7-phenyl-4-hepten-1-yl]morpholine.

32. A process for producing diphenylalkanoamine derivatives of the formula:

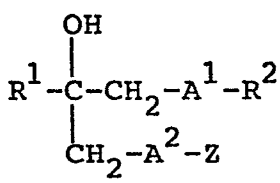

wherein $R^1$ and $R^2$ are each independently an aryl group optionally substituted with 1 to 2 substituents selected from the group consisting of halogen, $C_1$-$C_4$ alkyl, benzyloxy, hydroxyl, $C_1$-$C_4$ alkoxy, amino, $C_1$-$C_4$ alkylamino, di($C_1$-$C_4$)alkylamino and $C_1$-$C_4$ alkylthio, Z is a group of the formula:

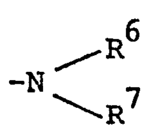

(wherein $R^6$ and $R^7$ are each independently a hydrogen atom or a $C_1$-$C_4$ alkyl group or, when taken together with the adjacent nitrogen atom to which they are attached, represent piperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino), $A^1$ is a $C_1$-$C_5$ alkylene group and $A^2$ is a $C_2$-$C_5$ alkylene group, and their non-toxic, pharmaceutically acceptable acid addition salts, which comprises reacting a compound of the formula:

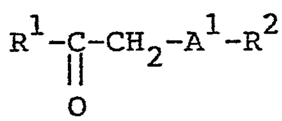

wherein $R^1$, $R^2$ and $A^1$ are each as defined above with a Grignard reagent of the formula:

$$X-Mg-CH_2-A^2-Z'$$

wherein X is a chlorine atom or a bromine atom and Z' is a group of the formula:

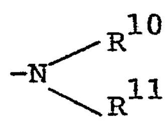

(wherein $R^{10}$ and $R^{11}$ are each independently a benzyl group or a $C_1$-$C_4$ alkyl group or, when taken together with the adjacent nitrogen atom to which they are attached, represent benzylpiperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino) and $A^2$ is as defined above and optionally subjecting the resulting

product to benzylation of the hydroxyl group and/or debenzylation of the benzyloxy, benzylamino, dibenzylamino or benzylpiperazino group.

33. A process for producing diphenylalkanoamine derivatives of the formula:

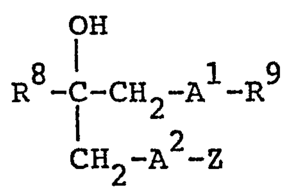

wherein $A^1$, $A^2$ and Z are each as defined in claim 32 and $R^8$ and $R^9$ are each independently an aryl group optionally substituted with 1 to 2 substituents selected from the group consisting of halogen, $C_1$-$C_4$ alkyl, benzyloxy, $C_1$-$C_4$ alkoxy, di($C_1$-$C_4$)alkylamino and $C_1$-$C_4$ alkylthio, and their non-toxic, pharmaceutically acceptable acid addition salts, which comprises reacting a compound of the formula:

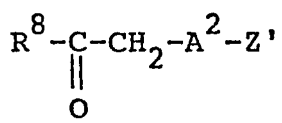

wherein $R^8$, $A^2$ are each as defined above and Z' is a group of the formula:

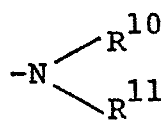

(wherein $R^{10}$ and $R^{11}$ are each independently a benzyl group or a $C_1$-$C_4$ alkyl group or, when taken together with the adjacent nitrogen atom to which they are attached, represent benzylpiperazino, piperidino, pyrrolidino, morpholino, hexamethyleneimino, $C_1$-$C_4$ alkylpiperidino, arylpiperidino,

ar($C_1$-$C_4$)alkylpiperidino, N-($C_1$-$C_4$)alkylpiperazino, N-arylpiperazino or N-ar($C_1$-$C_4$)alkylpiperazino) with a Grignard reagent of the formula:

$$X\text{-}Mg\text{-}CH_2\text{-}A^1\text{-}R^9$$

wherein X is a chlorine atom or a bromine atom and $R^9$ and $A^1$ are each as defined above and optionally subjecting the resulting product to debenzylation of the benzylamino, dibenzylamino or benzylpiperazino group.

34. A process for producing diphenylalkanoamine derivatives of the formula:

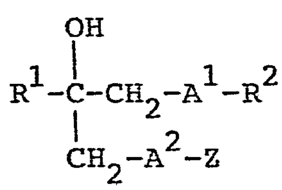

wherein $R^1$, $R^2$, $A^1$, $A^2$ and Z are each as defined in claim 32, and their non-toxic pharmaceutically acceptable acid addition salts, which comprises reacting a compound of the formula:

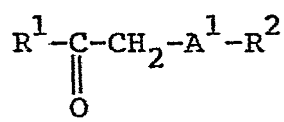

wherein $R^1$, $R^2$ and $A^1$ are each as defined above with a compound of the formula:

$$LiCH_2\text{-}A^2\text{-}Z'$$

wherein $A^2$ is as defined above and Z' is a group of the formula:

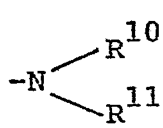

(wherein $R^{10}$ and $R^{11}$ are each independently a benzyl group or a $C_1$-$C_4$ alkyl group or, when taken together with the adjacent nitrogen atom to which they are attached, represent benzylpiperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino) and optionally subjecting the resulting product to benzylation of the hydroxyl group and/or debenzylation of the benzyloxy, benzylamino, dibenzylamino or benzylpiperazino group.

35. A process for producing diphenylalkanoamine derivatives of the formula:

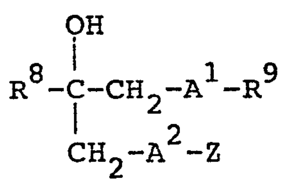

wherein $R^8$, $R^9$, $A^1$, $A^2$ and Z are each as defined in claim 33, and their non-toxic, pharmaceutically acceptable acid addition salts, which comprises reacting a compound of the formula:

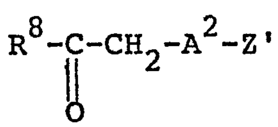

wherein $R^8$, $A^2$ are each as defined above and Z' is a group of the formula:

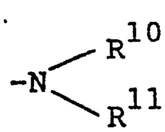

(wherein $R^{10}$ and $R^{11}$ are each independently a benzyl group or a $C_1$-$C_4$ alkyl group or, when taken together with the adjacent nitrogen atom to which they are attached, represent benzylpiperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino) with a compound of the formula:

$$LiCH_2-A^1-R^9$$

wherein $A^1$ and $R^9$ are each as defined above and optionally subjecting the resulting product to debenzylation of the benzylamino, dibenzylamino or benzylpiperazino group.

36. A process for producing diphenylalkanoamine derivatives of the formula:

$$\begin{array}{l} R^1-CH-CH_2-A^1-R^2 \\ \quad\;\; | \\ \quad\;\; CH_2-A^2-Z \end{array}$$

wherein $R^1$, $R^2$, $A^1$, $A^2$ and Z are each as defined in claim 32, and their non-toxic, pharmaceutically acceptable acid addition salts, which comprises reducing a compound of the formula:

$$\begin{array}{l} R^1-C-CH_2-A^1-R^2 \\ \quad\;\; \| \\ \quad\;\; CH-A^2-Z \end{array}$$

wherein $R^1$, $R^2$, $A^1$, $A^2$ and Z are each as defined above.

37. A process for producing diphenylalkanoamine derivatives of the formula:

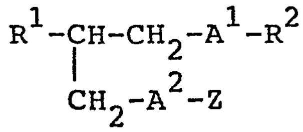

wherein $R^1$, $R^2$, $A^1$, $A^2$ and Z are each as defined in claim 32, and their non-toxic, pharmaceutically acceptable acid addition salts, which comprises reducing a compound of the formula:

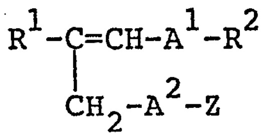

wherein $R^1$, $R^2$, $A^1$, $A^2$ and Z are each as defined above and optionally subjecting the resulting product to benzylation of the hydroxyl group and/or debenzylation of the benzyloxy or benzylpiperazino group.

38. A process for producing diphenylalkanoamine derivatives of the formula:

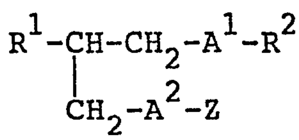

wherein $R^1$, $R^2$, $A^1$, $A^2$ and Z are each as defined in claim 32, and their non-toxic, pharmaceutically acceptable acid addition salts, which comprises dehydrating a compound of the formula:

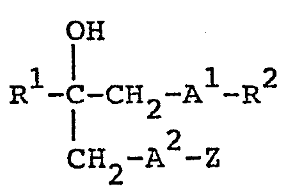

wherein $R^1$, $R^2$, $A^1$, $A^2$ and Z are each as defined above and reducing the dehydrated mixture of the compounds:

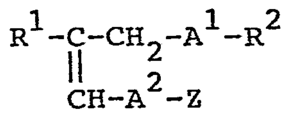

+

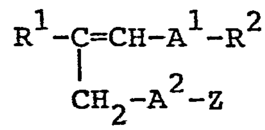

wherein $R^1$, $R^2$, $A^1$, $A^2$ and Z are each as defined above, optionally followed by subjecting the resulting product to benzylation of the hydroxyl group and/or debenzylation of the benzyloxy or benzylpiperazino group.

39. A process for producing diphenylalkanoamine derivatives of the formula:

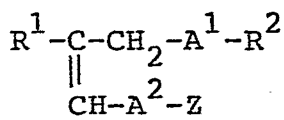

wherein $R^1$, $R^2$, $A^1$, $A^2$ and Z are each as defined in claim 32, and their non-toxic, pharmaceutically acceptable acid addition salts, which comprises reducing a compound of the formula:

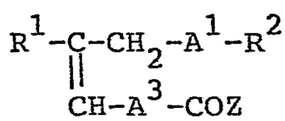

wherein $R^1$, $R^2$, $A^1$ and Z are each as defined above and $A^3$ is a $C_1$-$C_4$ alkylene group and optionally subjecting the resulting product to demethylation of the methylamino, dimethylamino or N-methylpiperazino group.

40. The process according to claim 39, wherein the starting compound is the one prepared by reacting a compound of the formula:

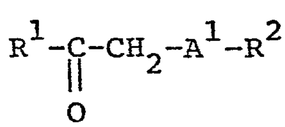

wherein $R^1$, $R^2$ and $A^2$ are each as defined above with a Wittig reagent of the formula:

$$(C_6H_5)_3P{=}CH{-}A^3{-}COOH$$

wherein $A^3$ is a $C_1$-$C_4$ alkylene group and subjecting the resulting compound of the formula:

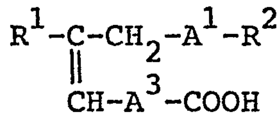

wherein $R^1$, $R^2$, $A^1$ and $A^3$ are each as defined above to reaction with a compound of the formula:

HZ

wherein Z is a group of the formula:

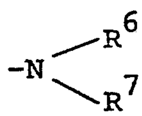

(wherein $R^6$ and $R^7$ are each independently a hydrogen atom or a $C_1$-$C_4$ alkyl group or, when taken together with the adjacent nitrogen atom to which they are attached, represent piperazino, piperidino, pyrrolidino, morpholino, hexamethyleneimino, $C_1$-$C_4$ alkylpiperidino, arylpiperidino, ar($C_1$-$C_4$)alkylpiperidino, N-($C_1$-$C_4$)alkylpiperazino, N-arylpiperazino or N-ar($C_1$-$C_4$)alkylpiperazino).

41. A process for producing diphenylalkanoamine derivatives of the formula:

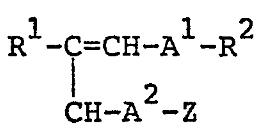

wherein $R^1$, $R^2$, $A^1$, $A^2$ and Z are each as defined in claim 32, and their non-toxic, pharmaceutically acceptable acid

addition salts, which comprises reacting a compound of the formula:

$$R^1-\underset{\underset{O}{\|}}{C}-CH_2-A^2-Z$$

wherein $R^1$, $A^2$ and Z are each as defined above with a Wittig reagent of the formula:

$$(C_6H_5)_3P{=}CH-A^1-R^2$$

wherein $R^2$ and $A^1$ are each as defined above and optionally subjecting the resulting product to demethylation of the N-alkyl-N-methylamino, dimethylamino or N-methylmorpholino group.

42. A pharmaceutical composition which comprises as an active ingredient a pharmaceutically effective amount of at least one of the compounds claimed in claim 1 and at least one pharmaceutically acceptable inert carrier or diluent.

43. Use of the compounds claimed in claim 1 as active ingredients for the preparation of a pharmaceutical composition for treating cerebral or coronary arteriosclerosis, senile mental indolence and the results of cerebral insufficiency.